# EUROPÄISCHE PATENTSCHRIFT

(11) **EP 1 973 498 B1**
(45) Veröffentlichungstag und Bekanntmachung des Hinweises auf die Patenterteilung: **23.04.2014**
(21) Anmeldenummer: 06819383.8
(22) Anmeldetag: 09.11.2006
(51) Int. Cl.: A61F 2/40, A61F 2/30, A61F 2/44, A61F 2/32

(54) **IMPLANTAT**
IMPLANT
IMPLANT

(30) Priorität: 09.11.2005 CH 182005
(43) Veröffentlichungstag der Anmeldung: 01.10.2008
(73) Patentinhaber: Zimmer GmbH, 8404 Winterthur (CH)
(72) Erfinder: SEEBECK, Jörn, CH-8483 Kollbrunn (CH); SIGGELKOW, Eik, CH-8600 Dübendorf (CH)
(74) Vertreter: Manitz, Finsterwald & Partner GbR
(86) Internationale Anmeldenummer: PCT/EP2006/068318
(87) Internationale Veröffentlichungsnummer: WO 2007/054553

(56) Entgegenhaltungen:
- EP-A- 0 013 864
- EP-A- 0 577 529
- DE-A1- 19 803 183
- DE-B- 1 164 019
- FR-A- 2 691 355
- US-A1- 2003 153 918
- US-B1- 6 168 630

## Beschreibung

Die Erfindung betrifft ein Implantat gemäss dem Oberbegriff des Anspruchs 1. Komponenten von Gelenksimplantaten werden üblicherweise an Resektionsflächen von Knochen befestigt. Auf Grund der natürlich vorgegebenen Kinematik der Gelenke werden die Verbindungsstellen, an denen die Komponenten der Implantate am Knochen befestigt sind, häufig parallel zur Verbindungsebene belastet und müssen teilweise auch Zug- oder Kippbelastungen, welche die Implantatkomponente vom Knochen zu trennen versuchen, aufnehmen können.

Derartige Belastungsformen treten beispielsweise bei der Tibiakomponente eines Kniegelenks auf.

Es ist aus dem Stand der Technik bekannt, beispielsweise zentrale Schäfte in den Markraum von Röhrenknochen einzuführen, oder die Komponente mit Zapfen zu versehen, welche die Fixierung der Komponente auf dem Knochen gegen Querbelastung aufnehmen. Bei Glenoidkomponenten von Schultergelenksprothesen ist es bekannt, diese zu verschrauben oder mit einzelnen Zapfen im Knochen zu befestigen.

DE 198 03 183 (Basis für den Oberbegriff des Anspruchs 1) beschreibt eine Tibiakomponente sowie eine Femurkomponente einer Kniegelenks-Totalprothese, welche an ihren dem Knochen zugewandt zur Implantation vorgesehenen Seiten konische Bohrungen aufweisen, in denen Befestigungsstifte zur Befestigung des Knochens im Implantat angeordnet werden können. Gemäss der dort vermittelten Lehre werden die Stifte so positioniert, wie es bei der jeweils vorliegenden Knochenstruktur am geeignetsten ist. Eine weitergehende Konkretisierung dieser Lehre vermittelt die DE 198 03 183 nicht, und vermittelt auch keine unmittelbar für den Fachmann umsetzbare Lehre, welche Positionierung der Stifte bei welcher Knochenstruktur geeignet sei.

EP 577 529 gibt ein Implantat an, welches zur Befestigung am Knochen eine Mehrzahl vereinzelt angeordneter stiftförmiger Verankerungselemente aufweist.

EP 013 864 vermittelt die Lehre, ein Implantat mittels einiger Verankerungszapfen mit einem wellenartigen Aussenprofil im Knochen zu verankern.

DE 1 164 019 gibt eine Kappe zum Ersatz der Gelenksfläche eines Femurkopfes an, die mittels drei Stiften zu verankern ist. Die Mehrzahl von Stiften dient dabei als Verdrehsicherung des kappenförmigen Implantats. Das Dokument betont für die dort vermittelte Lehre die Bedeutung der Verankerung der Stifte im kortikalen Knochengewebe des lateralen Femur und/oder des Adam'schen Bogens.

Die Erfindung geht davon aus, ein Implantat der eingangs genannten Art mit stiftförmigen Verankerungselementen zu versehen, welche in das Knochenmaterial einbringbar sind und dort Knochengewebe verdrängen. Insbesondere findet eine Mehrzahl von Verankerungsstiften Anwendung, welche insbesondere als Stiftfeld oder in einer Mehrzahl von Stiftfeldern angeordnet sind. Das Implantat findet den Halt also insbesondere nicht durch die Fixierung mittels einzelner Befestigungselemente, sondern durch das Zusammenwirken der Gesamtheit der Stifte, die sozusagen als Nagelbrett angeordnet sind. Die Kraft, die zum Verankern des Implantats erforderlich ist, wird somit im Wesentlichen auf die gesamte Resektionsfläche verteilt. Besonders bei schlechtem und insbesondere osteoporotischem Knochengewebe wird dadurch auch eine lokal starke Belastung von Knochengewebe vermieden und die einzelnen Bereiche des Knochens, in denen die Krafteinleitung stattfindet, sind im Wesentlichen über die gesamte Resektionsfläche und insbesondere über den spongiösen Bereich der Resektionsfläche verteilt, wodurch der Knochen im gesamten Bereich der Resektionsfläche eine Stimulation zum Knochenwachstum erhält. Durch die grossflächig verteilte Wachstumsstimulation wird insbesondere eine positive Wirkung auf die Knochenqualität im gesamten Bereich der Verankerung erzielt.

In unterschiedlichen Bereichen der Befestigungsseite des Implantats ist die Anordnung der Stifte und/oder die Geometrie der Stifte unterschiedlich gewählt. Unter der Anordnung der Stifte ist dabei die Position der Stifte auf der Befestigungsseite relativ zueinander zu verstehen, das heisst, beispielsweise der Abstand der Stifte voneinander und die Anzahl der Stifte pro Flächeneinheit der Befestigungsseite des Implantats. Als Geometrie der Stifte ist hier im weitesten Sinne sowohl die Länge als auch die Querschnittsfläche, die Formgebung des Querschnittes, und die Gestaltung der Stifte in ihrer Längserstreckung zu verstehen. Die Geometrie der Stifte wird bevorzugt so gewählt, dass diese an sich ohne Vorbohren des Knochens in diesen eingebracht werden können. Beim Eindringen in das Knochengewebe verdrängen die Stifte Knochengewebe und werden dadurch gehalten. Das gesamte Knochengewebe besteht aus knöchernem Gewebe und einem Markanteil. Aus Gründen der Einfachheit wird hier und im Folgenden auch der Markanteil des Knochens unter das Knochengewebe subsumiert, obschon es an sich nicht ganz präzise ist, den Markanteil des Knochens als Gewebe zu bezeichnen. Das knöcherne Gewebe umfasst kortikales Knochengewebe und spongiöses Knochengewebe. Die hier als knöchernes Gewebe zusammengefassten Bestandteile des Knochens weisen, im Gegensatz zum Mark, eine Struktur auf und sind daher in der Lage, eine Kraft zu übertragen. Sowohl die Festigkeit der Verankerung der Verankerungsstifte im Knochen als auch die Kraft, welche zum Einbringen der Verankerungsstifte in den Knochen erforderlich ist, hängen im Wesentlichen davon ab, wie viel knöchernes Gewebe, also kortikales Knochengewebe und an der Resektionsfläche eines Knochens insbesondere spongiöses Knochengewebe von einem Verankerungsstift verdrängt wird. Bei dem hier beschriebenen Implantat wird daher die Länge der Verankerungsstifte und/oder die Querschnittsfläche der Verankerungsstifte und/oder und die Anzahl der Verankerungsstifte je Flächeneinheit der Befestigungsseite in Abhängigkeit von der Dichte des Knochens, dem gegenüberliegend ein Flächensegment des Implantats vorgesehenen ist, das heisst, in Abhängigkeit vom Anteil des knöchernen Gewebes am Gesamtgewebe, unterschiedlich gewählt. Beispielsweise werden in Bereichen eines relativ geringeren Anteils knöchernen Gewebes am gesamten Gewebe die Verankerungsstifte länger gewählt und/oder es werden Verankerungsstifte mit einer grösseren Querschnittsfläche gewählt und/oder es werden Verankerungsstifte einer anderen Querschnittsform gewählt und/oder es werden mehr Stifte je Flächeneinheit der Befestigungsseite angeordnet. Gesamthaft gesehen wird also das Volumen der Verankerungsstifte je Flächeneinheit der Befestigungsseite des Implantats umso grösser gewählt, je kleiner der Anteil des knöchernen Gewebes am gesamten Knochengewebe ist. Die Geometrie und/oder die Anordnung der Stifte wird demnach entsprechend dem Anteil an knöchernem Gewebe am Gesamtgewebe des Knochenmaterials, dem gegenüberliegend der Bereich der Befestigungsseite vorgesehen ist, derart gewählt, dass in Bereichen eines relativ höheren Anteils knöchernen Gewebes gesamthaft weniger Gewebe, also knöchernes Gewebe und Mark, verdrängt wird als in Bereichen eines relativ geringeren Anteils knöchernen Gewebes. In einer Ausführungsform eines Implantats sind Geometrie und/oder die Anordnung der Verankerungsstifte derart gewählt, dass sich das Volumen der Verankerungsstifte je Flächeneinheit der Befestigungsseite im Wesentlichen umgekehrt proportional zum Anteil an knöchernem Gewebe im Gesamtgewebe des Knochens verhält, dem gegenüberliegend das Implantat vorgesehen ist. Dadurch wird in dem Knochen, dem gegenüberliegend das Implantat vorgesehen ist, an jeder Stelle im Wesentlichen gleich viel knöchernes Gewebe je Flächeneinheit der Befestigungsseite des Implantats verdrängt. Damit verteilt sich die Krafteinleitung der Verankerungsstifte gleichmässig auf die kraftübertragenden strukturierten Knochenbestandteile, derart, dass in Bereichen eines geringen Anteils an knöchernem Gewebe gesamthaft wieder mehr knöchernes Gewebe zur Kraftübertragung herangezogen wird. In Bereichen, in denen anteilig wenig knöchernes Gewebe vorhanden ist, in denen, mit anderen Worten, die Knochensubstanz wenig widerstandsfähig ist, wird die Krafteinleitung demnach über ein grösseres Volumen verteilt und die lokale Belastung des Gewebes wird somit reduziert.

In einer Ausführungsform des Implantats sind die Verankerungsstifte so angeordnet, dass sie bei der Implantation nur oder im Wesentlichen nur in die Spongiosa eindringen und somit durch Verdrängung der Trabekel eine Primärfixation erhalten, während nach einer gewissen Zeit ein Anwachsen von Knochengewebe an die Stifte erfolgt. Im Bereich des kortikalen Knochens werden in dieser Ausführungsform keine oder nur vergleichsweise kurze, d.h. höchstens einige wenige mm, zum Beispiel bis 2 oder 3 oder 5 mm lange stiftartige Verankerungselemente vorgesehen.

In einer Ausführungsform eines Implantats der beschriebenen Art sind in dem Bereich des Implantats, welcher zur Anordnung auf einem spongiösen Bereich der Resektionsfläche vorgesehen ist, die Geometrie und/oder die Anordnung der Verankerungsstifte neben der Abhängigkeit von dem Anteil des knöchernen Gewebes am gesamten Knochengewebe auch in Abhängigkeit von der Orientierung der Trabekel gewählt. In einer weiteren Ausführungsform sind die Verankerungsstifte nur in Bereichen des Implantats angeordnet, welche zur Anordnung auf einem spongiösen Bereich der Resektionsfläche vorgesehen sind, während in dem Bereich, der zur Anordnung auf der Kortikalis vorgesehen ist, keine oder allenfalls sehr kurze Stifte, eigentliche Spitzen, angeordnet sind. Insofern zeichnet sich einer Ausführungsform des Implantats dadurch aus, dass die Länge der Verankerungsstifte und/oder die Anzahl der Verankerungsstifte pro Flächeneinheit der Befestigungsseite des Implantats und/oder die Querschnittsfläche der einzelnen Verankerungselemente vom Rand der Befestigungsseite des Implantats zur Mitte der Flächen hin zunehmen.

Als Verankerungsstifte kommen beispielsweise Stifte in Frage, welche über ihre gesamte Längserstreckung einen konstanten Querschnitt aufweisen, insbesondere zylindrische Stifte, Stifte, welche sich zur Spitze hin in ihrer Querschnittsfläche verkleinern, insbesondere konische Stifte, sowie Stifte, welche in ihrer Längserstreckung Bereiche konstanten Querschnitts wie auch Bereiche variablen Querschnitts aufweisen. Stifte, welche einen sich verjüngenden Querschnitt aufweisen, weisen in diesem Bereich einen Halbwinkel auf, der im Maximum 5°, 4°, 3° oder 2° beträgt. Der Winkel ist beispielsweise klein genug, um einen wenigstens näherungsweise selbsthemmenden Sitz des Verankerungsstiftes in dem Knochenmaterial zu gewährleisten. Die Geometrie des Querschnitts der Verankerungsstifte ist beispielsweise kreisförmig, kann aber ohne weiteres auch ein Polygon sein, insbesondere dreieckig oder rechteckig, oder kann Kreuzform oder Sternform aufweisen, oder kann auch ein Hohlprofil sein, wobei diese Ausgestaltung ohne weiteres bei den Verankerungsstiften, die in verschiedenen Bereichen der Befestigungsseite eines Implantats angeordnet sind oder bei verschiedenen Verankerungsstiften, unterschiedlich sein kann. Die Grösse des Querschnitts der Verankerungsstifte kann dann durch einen Durchmesser eines dem Stiftquerschnitt an der Basis des Stiftes umbeschriebenen Kreises angegeben werden, und liegt beispielsweise im Bereich von 0,5 Millimeter bis 3 Millimeter.

Eine Ausführungsform des Implantats zeichnet sich dadurch aus, dass die Verankerungsstifte hinsichtlich ihrer Länge und/oder hinsichtlich der Anzahl der Stifte je Flächeneinheit der Befestigungsseite und/oder hinsichtlich ihrer Querschnittsfläche variieren.

In einer anderen Ausführungsform sind an den dem Implantat benachbarten Enden der Verankerungsstifte wenigstens zwei Verankerungsstifte durch eine wandartige Struktur miteinander verbunden, welche sich einerseits von einem Verankerungsstift zum anderen Verankerungsstift erstreckt und sich andererseits um eine Höhe von der Befestigungsseite zum abgewandten Ende der Verankerungsstifte hin erstreckt, wobei die Höhenerstreckung kleiner als die Länge der Verankerungsstifte ist und wobei in einer spezifischeren Ausführungsform die Höhenerstreckung der wandartigen Struktur zwischen 1 mm und 4 mm beträgt. Diese Anordnung verbessert unter Anderem bei Bedarf die Sicherheit der Stifte gegen Knicken bei der Implantation, was aus dem Grunde wichtig ist, weil ja die Stifte bevorzugt ohne Vorbohren in den Knochen eingedrückt werden.

Weiterhin zeichnet sich ein Implantat dadurch aus, dass die Befestigung durch die Gesamtheit der Stifte bewirkt wird, die nach der Art eines Nagelbretts angeordnet sind. Dabei betragen in einer Ausführungsform die Abstände (s) zwischen den Längsachsen der Verankerungstifte wenigstens 1 mm, insbesondere 1,5 mm und weiterhin wenigstens 2 mm und nicht mehr als 10 mm, insbesondere nicht mehr als 5mm und weiterhin nicht mehr als 3 mm. Dabei beträgt die Flächendichte der Verankerungsstifte im Minimum rund 3 Stift/cm² und im Maximum 30/cm². Weiterhin sind in einer beispielhaften Ausführungsform des Implantats die Verankerungsstifte wenigstens bereichsweise auf einem äquidistanten Raster, insbesondere auf einem Grundraster aus gleichseitigen Dreiecken, angeordnet.

In einer Ausführungsform weist wenigstens ein Verankerungsstift einen zur Befestigungsseite des Implantats hin divergenten Abschnitt auf, wobei der Halbwinkel (α) der Divergenz im Maximum 5°, 4°, 3°, oder 2° beträgt. Ein derartiger spitzer Konus erleichtert einerseits das Einbringen der Verankerungsstifte in den Knochen und gewährleistet andererseits einen guten und zuverlässigen Halt der Stifte.

Beispielsweise zeichnet sich ein Implantat dadurch aus, dass die Querschnitte der Verankerungsstifte umbeschriebene Kreise aufweisen, deren Durchmesser an der Basis der Stifte, d.h. am der Befestigungsseite des Implantats zugewandten Ende, wenigstens 0,5 mm und insbesondere wenigstens 1 mm betragen. Weiterhin betragen diese Durchmesser in einer beispielhaften Ausführungsform an der Basis der Stifte höchstens 3 mm, insbesondere höchstens 2 mm und weiterhin insbesondere höchstens 1 mm. Die Länge eines längsten Verankerungsstiftes der Verankerungsstifte beträgt beispielsweise minimal 8 mm, insbesondere minimal 15 mm oder minimal 20 mm und in einer Ausführungsform minimal 25 mm. In einer weiteren Ausführungsform beträgt die Länge der Verankerungsstifte generell minimal 2 mm, insbesondere minimal 3 mm oder minimal 5 mm und in einer Ausführungsform minimal 10 mm. Die Maximallänge der Verankerungsstifte liegt in einer Ausführungsform des Implantats bei maximal 50 mm insbesondere maximal 35 mm und in einer speziellen Ausgestaltung maximal 25 mm. In einer Ausführungsform weist ein Implantat wenigstens einen Verankerungsstift mit einer Länge von wenigstens 15 mm und insbesondere wenigstens 25 mm auf.

Die Verankerungsstifte, weisen weiterhin insbesondere Längen-Durchmesser-Verhältnisse von wenigstens 3 auf, wobei in einer Weiterbildung wenigstens einige Verankerungsstifte Längen-Durchmesser-Verhältnisse von wenigstens 8 und insbesondere wenigstens 10 oder 12 aufweisen.

Die Längsachsen der Verankerungsstifte sind insbesondere im Wesentlichen parallel zueinander und weiterhin im Wesentlichen senkrecht zu einer dem Implantat zugeordneten Resektionsfläche angeordnet.

Generell ist ein Implantat der hier vorgeschlagenen Art besonders dann besonders vorteilhaft, wenn es zur Implantation auf einem Knochenbereich mit vergleichsweise schlechter Knochenqualität vorgesehen ist, die die Kraftübertragung über eine grosse Fläche verteilt wird, und eine Anpassung der Dichte des Kraftflusses an die lokale Knochenqualität erfolgt.

Weitere Kriterien für die Auslegung der Geometrie der Verankerungsstifte und deren Anordnung auf der Befestigungsseite des Implantats ergeben sich aus den Unteransprüchen und den Ausführungsbeispielen.

In einer Weiterbildung des Implantats ist wenigstens ein Führungs- und/oder Zentrierelement auf der Befestigungsseite angeordnet, welches sich insbesondere weiter von der Befestigungsseite weg erstreckt als der längste Verankerungsstift. Beim Aufsetzen des Implantats wird das Führungs- und/oder Zentrierelement in eine vorgebohrte Öffnung des Knochens eingeführt, und legt somit vor der eigentlichen Verankerung des Implantats mittels der Verankerungsstifte die Position des Implantats auf dem Knochen fest. In noch einer Weiterbildung des Implantats weist das Implantat zwei solcher Führungsund/oder Zentrierelemente auf. Diese legen dann die Position und die Orientierung des Implantats auf dem Knochen fest, bevor die Stifte in den Knochen eindringen und das Implantat fixieren.

Eine Ausgestaltung des oben beschriebenen Implantats ist ein proximales Tibiaimplantat, insbesondere ein Tibiaplateau, wie es beispielsweise für Kniegelenksprothesen Anwendung findet.

Eine weitere Ausgestaltung ist eine distale Femurkomponente einer Kniegelenksprothese. Dabei kann es sich um eine monokondyläre oder eine bikondyläre Femurkomponente handeln.

Eine andere Ausgestaltung ist eine proximale Femurkomponente für ein Hüftgelenk, insbesondere eine Kappe zur Anordnung auf dem Femurkopf, wie sie für das sogenannte "Resurfacing" Verwendung findet. In einer Ausführungsform weist die proximale Femurkomponente eine metallische Artikulationsfläche auf, welche beispielsweise einen Formfehler weniger als 10 µm oder gar weniger als 2 µm aufweist. Eine solche proximale Femurkomponente ist beispielsweise zur Verwendung mit einer Metall-Metall-Gleitpaarung in einer Hüftgelenksprothese geeignet.

Selbstverständlich ist das beschreibene Implantat auch als Acetabulumkomponente einer Hüftgelenksprothese geeignet.

Ebenso kann das Implantat als Bestandteil einer Schultergelenksprothese Verwendung finden, welches mittels der Verankerungsstifte an der Scapula oder am Humerus befestigt wird.

Eine andere Ausführungsform betrifft ein intervertebrales Implantat, welches mittels der Verankerungsstifte in den Wirbelkörpern verankert wird.

Diese Aufzählung kann und will nicht abschliessend sein.

Eine Ausführungsform des Implantats zeichnet sich dadurch aus, dass im Wesentlichen auf der gesamten der Resektionsfläche eines Knochens und insbesondere dem spongiösen Bereich der Resektionsfläche gegenüberliegend vorgesehenen Oberfläche vergleichsweise dicht beieinanderliegende Verankerungsstifte vorgesehen sind, deren Abstand voneinander nur wenige mm, beispielsweise bis 3 mm oder bis 5 mm, beträgt.

Die Verankerungsstifte gewährleisten eine gute Primärfixierung des Implantats im Knochen aufgrund der Haftreibung des verdrängten Knochenvolumens an der Vielzahl von Stiften. Die so erzielte Primärfixation ist umso grösser, je steiler die Seiten der Stifte sind. Weisen die Stifte jedoch eine ausgeprägte Kegelform auf, die sich mit einem vergleichsweise grossen Divergenzwinkel zur Basis der Stifte hin vergrössert, ist der Halt geringer und das Implantat kann bei allfälligen Folgeeingriffen leichter wieder entfernt werden. Ebenso können die Stifte mit unterschiedlicher Oberflächenbeschaffenheit ausgeführt sein, um die Sekundärfixation durch das Anwachsen von Knochen zu beeinflussen. Generell wird die Sekundärverankerung bei einer rauen Oberfläche ausgeprägter sein als bei vergleichsweise glatten Oberflächen. Auch eine Beschichtung mit Hydroxilapatit oder dergleichen ist möglich. Beispielsweise sind die Stifte aus Titan hergestellt.

Selbstverständlich können die oben und in den Ansprüchen beschriebenen Merkmale von Implantaten der hier beschriebenen Art auch untereinander kombiniert werden.

Ein Implantat der oben beschriebenen Art eignet sich zur Implantation unter Verwendung von Knochenzement wie auch zur zementfreien Implantation.

Das oben beschriebene Implantat wird nachfolgend anhand in der Zeichnung dargestellter Ausführungsbeispiele näher beschrieben. Im einzelnen zeigen
Figur 1 eine schematische Darstellung eines Tibiaimplantats der beschriebenen Art, sowie eine Draufsicht auf eine Resektionsfläche einer Tibia, zur Anordnung auf welche das Implantat vorgesehen ist;
Figur 2 eine weitere Ausführungsform des Implantats aus Figur 1;
Figur 3 eine Seitenansicht einer in der beschriebenen und beanspruchten Weise ausgeführten Femurkomponente einer Kniegelenksprothese;
Figur 4 eine schematische Darstellung einer Femurkopfprothese sowie eines zur Implantation vorbereiteten Femur;
Figur 5 verschiedene beispielhafte Geometrien von Verankerungsstiften;
Figur 6 ein Beispiel für die Anordnung von Stiften auf einem Raster;
Figur7 verschiedene beispielhafte Stiftquerschnitte;
Figur 8 eine Ausführungsform einer Anordnung von Verankerungsstiften;
Figur 9 eine weitere Ausgestaltung eines Tibiaimplantats sowie eine Draufsicht einer präparierten Tibia;
Figur 10 ein weiteres Beispiel eines Tibiaimplantats;
Figur 11 ein Tibiaimplantat aus Figur 10 im implantierten Zustand;
Figur 12 ein Schulterimplantat, bei dem die Humeruskomponente wie auch die Glenoidkomponente in der beschriebenen Art verankert sind;
Figur 13 eine erste Ansicht der Glenoidkomponente aus Figur 12;
Figur 14 eine zweite Ansicht der Glenoidkomponente aus Figur 12; und
Figur 15 die Humeruskomponente aus Figur 12.

Die Ausführungsbeispiele dienen dem besseren Verständnis der Erfindung und sollen nicht zu einer Einschränkung der in den Ansprüchen beschriebenen Erfindung herangezogen werden.

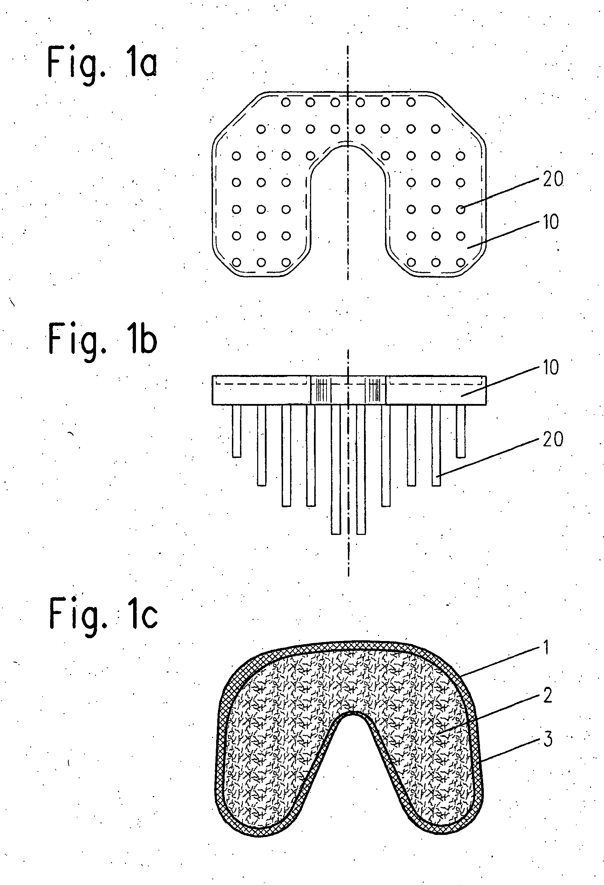

In der Figur 1 ist in einer stark vereinfachten Darstellung eine Tibiaplatte 10 gezeigt, welche in der vorgeschlagenen Art ausgeführt ist. Die Figur 1a zeigt eine Draufsicht auf die Befestigungsseite, mit welcher das Implantat zur Anlage auf der Resektionsfläche des Knochens vorgesehen ist. Auf der Befestigungsseite der Tibiaplatte 10 sind Verankerungsstifte 20 angeordnet. In der Figur sind diese auf einem quadratischen Raster angeordnet, was aber keinesfalls zwingend ist; auch beliebige andere der Anwendung angemessene Anordnungsmuster sind möglich. Figur 1b zeigt eine Ansicht entlang der in Figur 1a mit B-B gekennzeichneten Linie. Die Figur 1c zeigt eine Draufsicht auf die Resektionsfläche einer Tibia 1. Die Kortikalis 3 am Rand der Resektionsfläche. besteht im Wesentlichen aus festem Knochengewebe und ist nur wenig verformbar und vergleichsweise spröde. Im Bereich der Spongiosa 2 besteht das Knochengewebe, wie oben bereits beschrieben, aus knöchernem Gewebe und Mark und weist eine grössere Verformbarkeit auf. Wie durch die unterschiedlich dichten Schraffuren angedeutet, ist der Anteil an knöchernem Gewebe am Gesamtgewebe in verschiedenen Bereichen der Resektionsfläche unterschiedlich. In dem hier gezeigten Beispiel nimmt der Anteil des knöchernen Gewebes von der Kortikalis zur Mitte hin ab, wodurch auch die Festigkeit abund dir Verformbarkeit des Knochens zunimmt. Mit Bezug zur Figur 1b ist zu erkennen, dass die Länge der Verankerungsstifte diesem Umstand angepasst ist, und in den Bereichen, die zur Anordnung in Bereichen des Knochens mit einem geringen Anteil an knöchernem Gewebe vorgesehen sind, grösser ist als in Bereichen eines hohen Anteils an knöchernem Gewebe. In diesem Beispiel sind alle Verankerungsstifte als zylindrische Stifte mit konstanter Querschnittsfläche dargestellt. Selbstverständlich können die Stifte auch gesamthaft oder bereichsweise konisch oder beispielsweise am vorderen dem Implantat abgewandten Ende spitz zulaufend sein. Ebensowenig ist es zwingend, dass alle Stifte die gleiche Querschnittsform und Längskontur aufweisen.

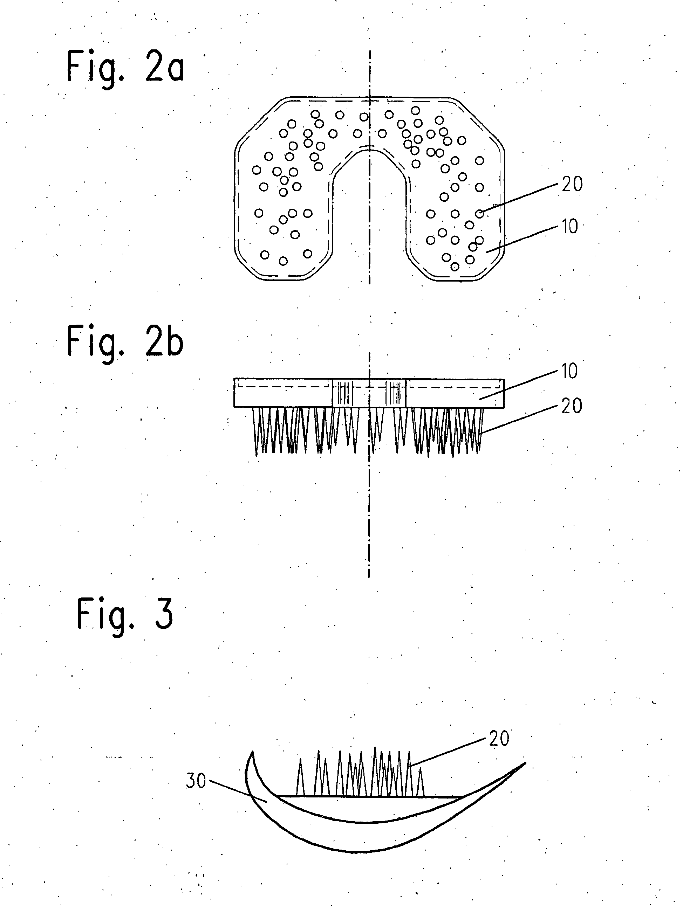

Die Figur 2 zeigt eine alternative Ausführungsform eines derartigen Tibiaplateaus. Auf dem Tibiaplateau 10 sind die Verankerungsstifte 20 in einer über der Fläche der Befestigungsseite des Implantats variablen Dichte angeordnet. Wie in der Figur 2b zu erkennen ist, sind die Verankerungsstifte 20 nahezu gleich lang und spitz zulaufend. Durch die Verringerung des Abstandes zwischen den Verankerungsstiften in den Bereichen des Implantats, welche zur Anordnung auf Bereichen einer Resektionsfläche mit geringerem Anteil an knöchernem Material vorgesehen sind, wird ein äquivalenter Effekt erreicht wie er sich in der Figur 1 aufgrund der längeren Verankerungsstifte einstellt. Selbstverständlich können auch bei der in der Figur 2 dargestellten Ausführungsform Verankerungsstifte unterschiedlicher Länge und anderer und/oder unterschiedlicher Formgestaltung Verwendung finden.

In der Figur 3 ist schematisch ein Kondylenimplantat 30 dargestellt, welches Verankerungsstifte 20 unterschiedlicher Länge zur Verankerung an der Resektionsfläche eines Knochen aufweist.

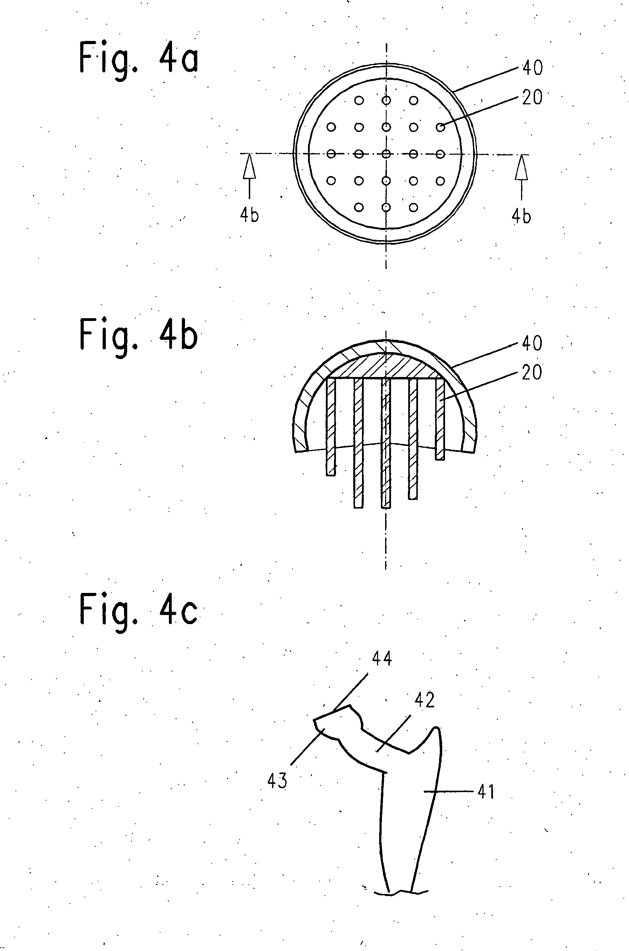

In der Figur 4 ist die Anwendung eines Implantats der hier beschriebenen Art an der Femurkomponente einer Hüftgelenksprothese dargestellt. In den Figuren 4a und 4b ist eine Femurkappe dargestellt, wie sie für das sogenannte Resurfacing eines Femurkopfes Anwendung findet. Das "Resurfacing" zeichnet sich dadurch aus, dass möglichst wenig Knochen abgetragen wird, und im Wesentlichen nur der Knorpel der natürlichen Artikulationsfläche durch eine künstliche Artikulationsfläche ersetzt wird. Die Artikulationsfläche der Femurkappe 40 besteht bevorzugt aus Metall, insbesondere aus einem Stahl mit einem hohen Kohlenstoffanteil, was hier aber nicht erfindungswesentlich ist. In der Figur 4c ist schematisch der Femur 41 dargestellt mit dem Femurhals 42, dem Femurkopf 43 und der Resektionsfläche 44 am Femurkopf. Auch über dieser Resektionsfläche ist der Anteil des knöchernen Gewebes am gesamten Knochengewebe unterschiedlich. Beispielsweise findet sich am äusseren Rand der Resektionsfläche Kortikalis, in welche Verankerungsstifte nur schwer einzubringen sind, und wobei auch das Risiko besteht, bei nicht vorgebohrten Löchern zum Einbringen der Stifte eine Splitterung der Kortikalis zu verursachen. Im Zentrum der Resaktionsfläche findet sich hingegen spongiöser Knochen mit einem grossen Markanteil. Daher sind die Verankerungsstifte 20 der Femurkappe 40 der Knochendichte angepasst in verschiedenen Bereichen unterschiedlich gewählt und im Zentrum länger als am Rand, wohingegen ganz am Rand, in Bereichen die auf der Kortikalis zu liegen kommen, keine Verankerungsstifte angeordnet sind. Die hier beschriebene Befestigung eines Implantats mittels eines Kollektivs von an die lokale Knochenstruktur und - festigkeit angepassten Stiften kann selbstverständlich auch bei der Befestigung der zugehörigen Acetabulumkomponente Anwendung finden.

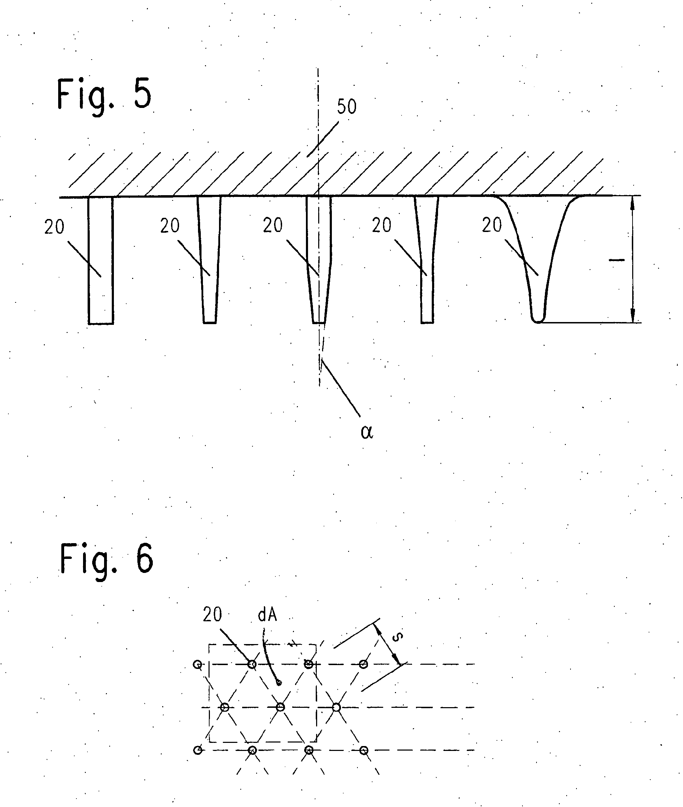

In der Figur 5 sind schematisch mehrere mögliche Ausführungsformen von Verankerungsstiften 20 dargestellt. Diese erstrecken sich ausgehend vom Implantat 50 mit einer Länge I. Einige der beispielhaften Verankerungsstifte 20 weisen von der Spitze zum Implantat hin gesehen divergente Bereiche auf. Der Halbwinkel der Divergenz ist mit α bezeichnet. Dieser Winkel liegt beispielhaft bei 2° ,3 °, 4°, oder 5°. Kleine Winkel, entsprechend sehr spitzen Stiften, erleichtern das Einbringen, das insbesondere in einen nicht vorgebohrten Knochen erfolgt, und verbessern die Sicherheit des Sitzes der Stifte im Knochen. Stifte mit grösseren Divergenzwinkeln hingegen weisen eine grössere Sicherheit gegen Knicken beim Einbringen auf und erleichtern die Explantation bei allfälligen Revisionen.

Die Figur 6 verdeutlicht weitere geometrische Zusammenhänge der Anordnung von Verankerungsstiften auf der Befestigungsseite eines Implantats. In diesem Beispiel sind die Verankerungsstifte 20 auf einem Raster gleichseitiger Dreiecke angeordnet. Die Abstände zwischen den zentralen Längsachsen zweier benachbarter Verankerungsstifte ist mit s bezeichnet. Die Dichte der Verankerungsstifte ist die Anzahl von Stiften innerhalb eines Flächenelementes dA, welche zum Beispiel als die Anzahl der Stifte auf einer Einheitsfläche von beispielsweise 1 cm² bezogen spezifiziert wird.

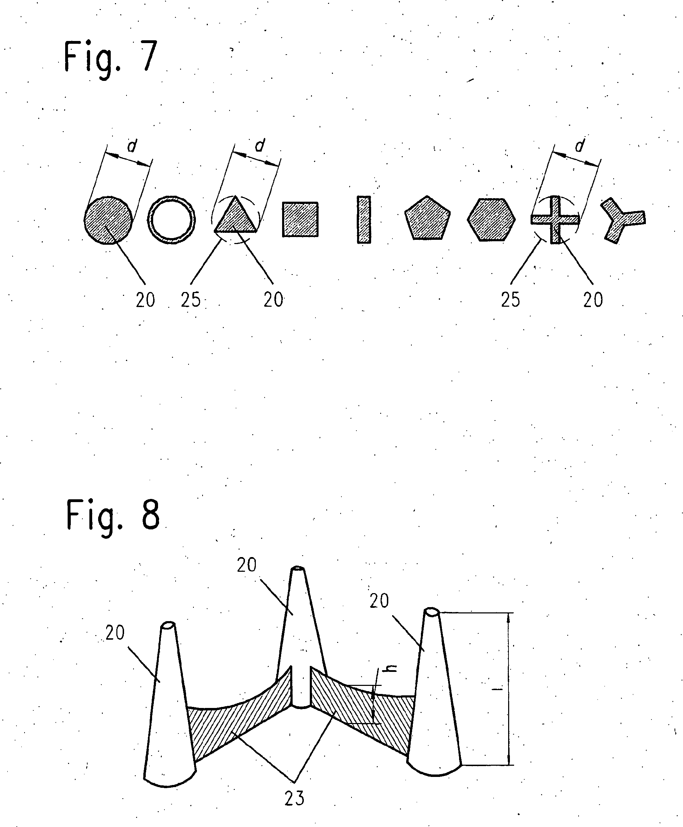

Die Figur 7 illustriert verschiedene Querschnittsformen von Verankerungsstiften. Weiterhin ist bei einigen dieser beispielhaften Verankerungsstifte der umbeschriebene Kreis 25 mit dem Durchmesser d dargestellt.

Figur 8 illustriert eine weitere Ausführungsform, bei der zwischen Verankerungsstiften 20 wandartige Strukturen 23 angeordnet sind, deren Höhe h kleiner als die Länge l der Verankerungsstifte 20 ist, wobei die Höhe innerhalb einer solchen wandartigen Struktur auch variabel sein kann, beispielsweise derart, dass die Höhe,der wandartigen Struktur benachbart zu den Verankerungsstiften grösser ist als in der Mitte zwischen zwei Verankerungsstiften; selbstverständlich sind auch wandartige Strukturen konstanter Höhe möglich, oder solche, bei denen die Höhe in der Mitte zwischen zwei Verankerungsstiften grösser ist als benachbart zu den Verankerungsstiften. Die Höhenerstreckung der wandartigen Struktur beträgt beispielsweise zwischen ein Millimeter und vier Millimeter, und sollte so beschränkt sein, dass nicht zu viele Blutgefässe innerhalb des Knochens durch diese wandartigen Strukturen zerschnitten werden.

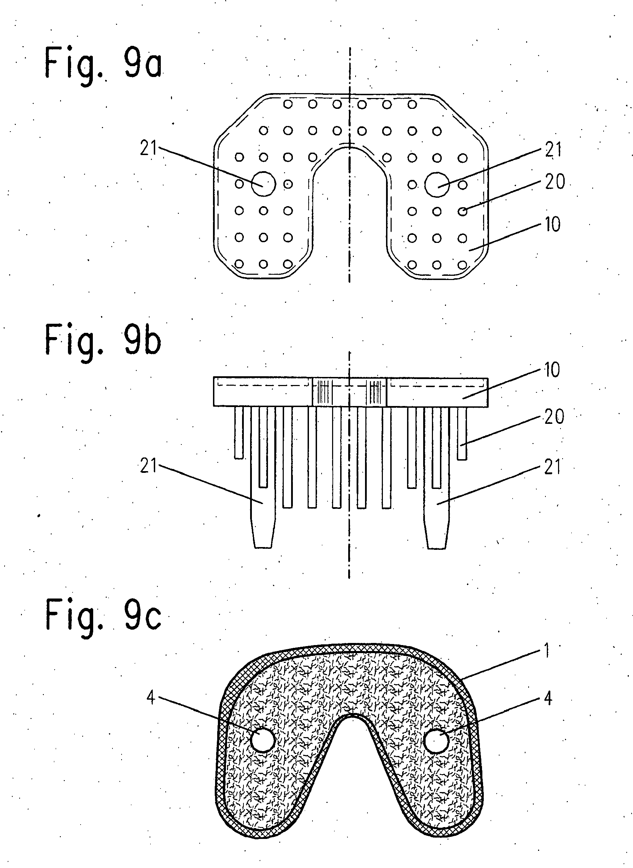

Die Figur 9 zeigt eine Abwandlung der Tibiaplatte aus der Figur 1. Die Tibiaplatte 10 weist auf der Befestigungsseite neben den Verankerungsstiften 20 Führungselemente 21 auf, die weiter aus der Befestigungsseite herausragen als der längste Verankerungsstift. In die in der Figur 9c dargestellte Resektionsfläche der Tibia sind zwei Positionierbohrungen 4 eingebracht, welche in ihrem Abstand dem Abstand der Führungselemente 21 entsprechen. Beim Aufsetzen der Tibiaplatte auf die Resektionsfläche dringen zunächst die Führungselemente 21 in die Bohrungen 4 der Tibia ein und positionieren dort die Tibiaplatte bezüglich Lage und Richtung, bevor das Implantat durch Eindrücken oder Einschlagen der Verankerungsstifte in das Knochengewebe im Knochen fixiert wird. Zum leichteren Einführen in die Bohrungen 4 weisen die Führungselemente 21 neben dem zylindrischen Führungsbereich einen konischen Spitzenbereich auf. Für eine bestmögliche Funktion ist der zylindrische Führungsbereich der Führungselemente länger als der längste Verankerungsstift, derart, dass Lage und Richtung des Implantats vor dem Eindringen der Verankerungsstifte bereits sicher festgelegt sind.

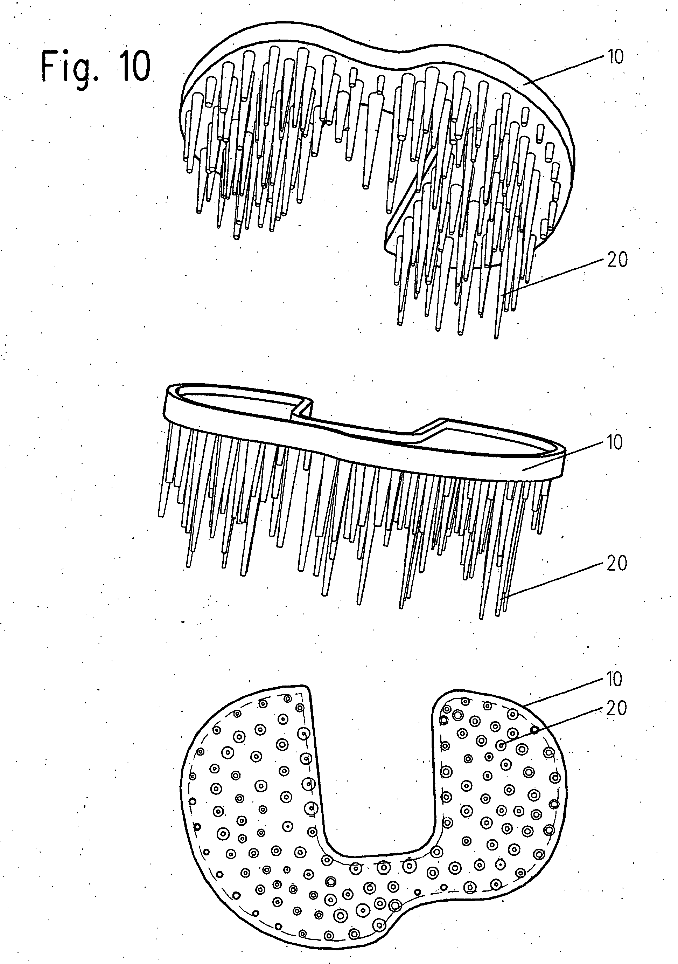

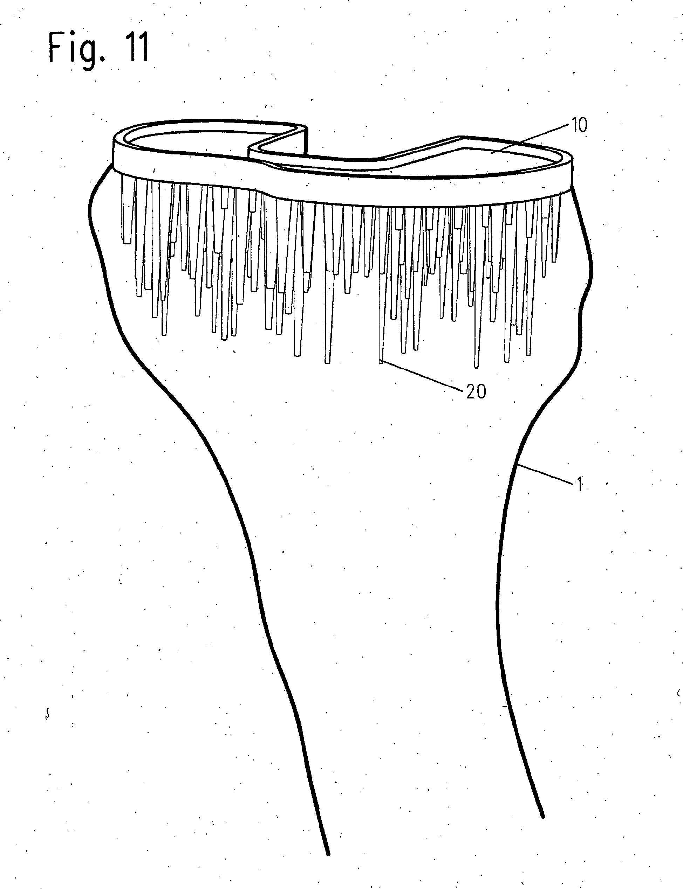

Die Figur 10 zeigt eine beispielhafte Tibiaplatte 10, bei der die Anordnung und Geometrie der Verankerungsstifte 20 der komplexen Knochendichteverteilung an einer realen Resektionsfläche einer Tibia angepasst ist. Die Figur 11 zeigt diese Tibiaplatte auf einer Tibia 1 im implantierten Zustand.

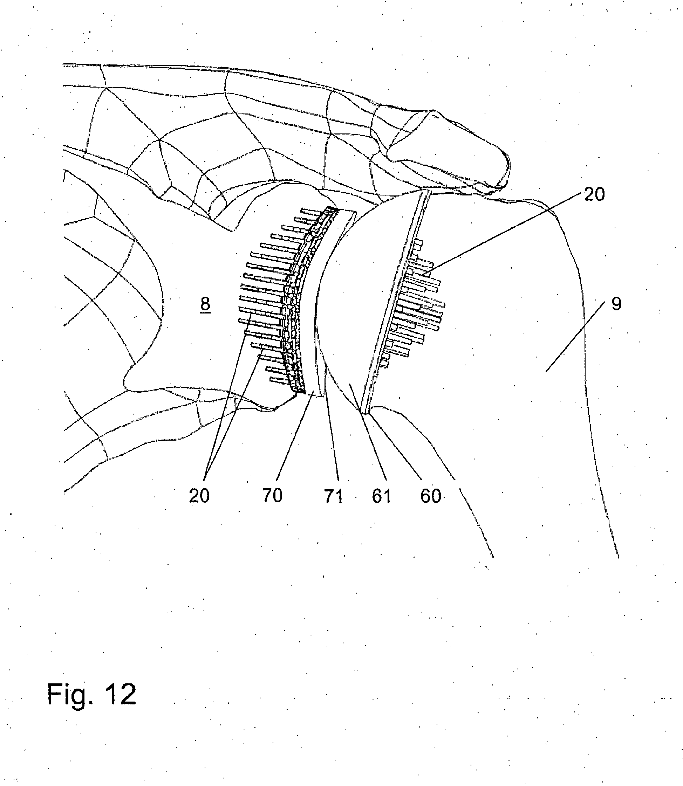

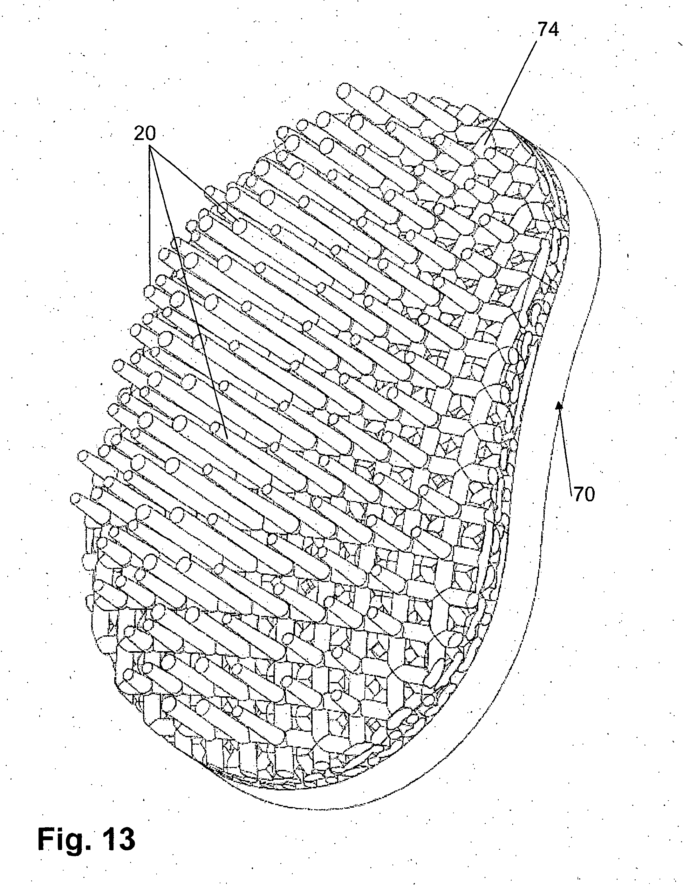

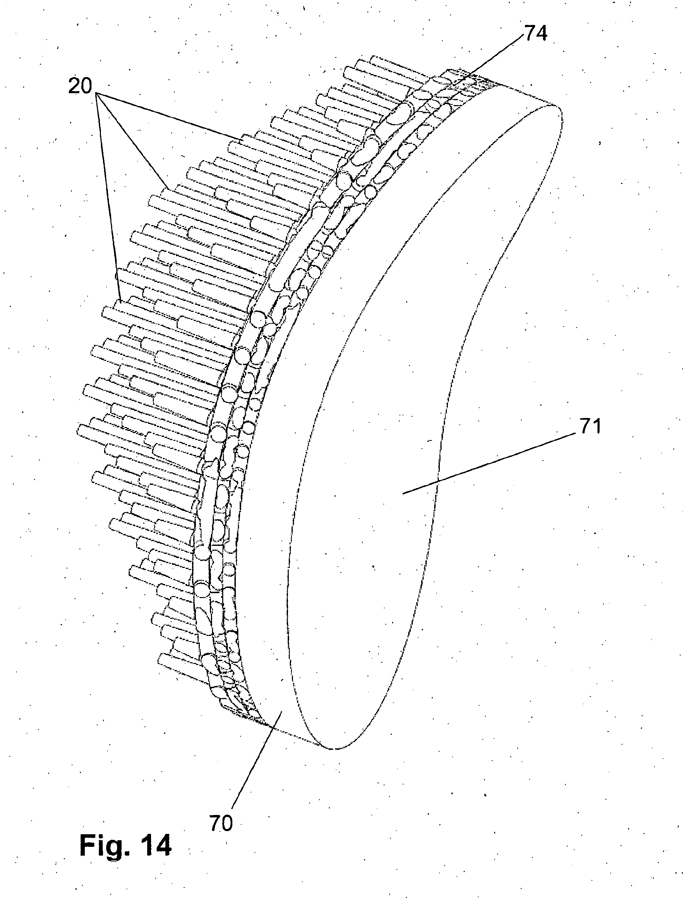

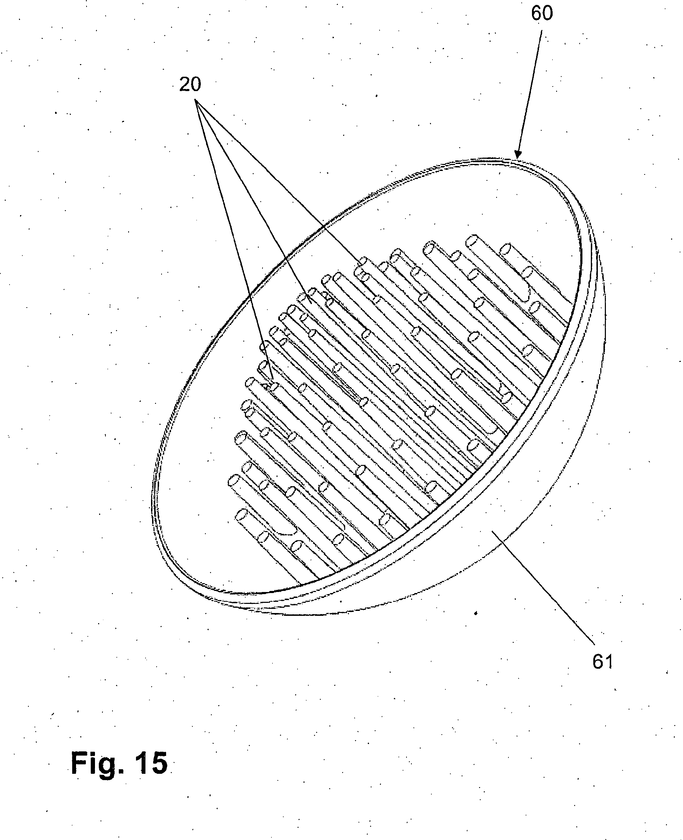

In der Figur 12 ist eine Schulterprothese dargestellt, bei welcher die Komponenten in der beschriebenen und beanspruchten Art ausgeführt sind. Eine Kappe 60, die beispilesweise aus einem Metallischen Werkstoff hergestellt ist, mit einer Artikulationsfläche 61 ist mittels Verankerungsstiften 20 unterschiedlicher Länge im Humerus 9 verankert. Beim Aufbringen einer solchen Kappe als Gelenksflächenersatz handelt es sich ebenfalls um ein eigentliches "Resurfacing" in der im Zusammenhang mit Figur 4 erläuterten Art. Eine Glenoidkomponente 70 mit einer Artikulationsfläche 71 ist ebenfalls mittels einer Mehrzahl von in einem Stiftfeld angeordneten Verankerungsstiften 20 in der Scapula 8 verankert. An diesen Stellen findet der Operateur häufig sehr schlechte Knochenqualität vor, welche eine sichere Primärfixation der Implantate erschwert. Die Verteilung der Verankerung auf die Vielzahl von Stiften, die derart geformt und angeordnet sind, dass die Kraft auf ein umso grösseres Knochenvolumen verteilt wird je geringer die lokale Dichte und Stabilität des Knochens ist, kommt sehr vorteilhaft zum Tragen. Durch die oft vorgefundene starke Porosität des Knochens an den Implantationsstellen wird die Implantation durch Eindrücken oder Einschlagen der Implantate vereinfacht, während die Verteilung der Haltekräfte auf viele Stifte die Primärfixation erleichtert. Für eine sekundäre Fixation durch Anwachsen des Knochens steht eine grosse Oberfläche der Stifte 20 zur Verfügung. Die Festigkeit des Halts und die Intensität der Verbindung mit dem Knochen kann durch die Geometrie der Stifte, wie oben beschrieben, und deren Oberflächenbeschaffenheit, insbesondere deren Rauhigkeit, auf an sich bekannte Weise beeinflusst werden. Die Glenoidkomponente ist in den Figuren 13 und 14 vergrössert dargestellt. Sie umfasst in diesem Beispiel neben den Verankerungsstiften 20 eine Artikulationsfläche 71, welche beispielsweise aus Polyethylen, insbesondere aus einem hochvernetzten und/oder einem hochmolekularen Polyethylen, besteht, sowie ein mehrlagiges Drahtgitter, welches beispielsweise aus Titan besteht und beispielsweise unter dem Namen "Sulmesh" bekannt ist. Dieses eignet sich gut zur Verankerung der Polyethylen-Artikulationsfläche und begünstigt ebenfalls ein Anwachsen des Knochens. Figur 15 zeigt die Humeruskomponente, welche hier die Form einer Hohlkalotte 60 aufweist, und in deren Innerem die Verankerungsstifte 20 angeordnet sind. Sowohl bei der Glenoidkomponente 70 als auch bei der Humeruskomponente 60 sind die Stifte im Zentrum der Verankerungsfläche länger als an deren Rand.

Die oben beschriebenen Ausführungsformen stellen nur einige ausgewählte Beispiele aus den Möglichkeiten dar, welche die Erfindung dem Fachmann zur Verfügung stellt. Weitere Ausführungsformen der Erfindung erschliessen sich dem Fachmann im Lichte dieser Ausführungen ohne Weiteres.

Die Implantation der hier beschriebenen Implantate erfolgt, indem der entsprechende Knochen geschnitten wird. Die Verankerungsstifte werden in die Knochenstruktur der entstehenden Resektionsfläche eingedrückt, ohne dass entsprechende Löcher vorgebohrt wurden. Bei der Verwendung von Implantaten mit Zentrier- und/oder Führungszapfen, wie es in der Figur 9 dargestellt ist, werden Bohrungen zur Aufnahme der Zapfen und den Knochen eingebracht; Bohrungen zur Aufnahme der Stifte werden nicht benötigt. Die Reibung der Stifte im Kochengewebe und die grosse Anzahl der Stifte, sowie deren Anpassung - sei es durch die Anordnung oder die Geometrie - an die lokale Knochendichte gewährleisten eine gute Primärverankerung des Implantats im Knochen, so, dass die Implantation zementfrei erfolgen kann; selbstverständlich ist es auch möglich, das Implantat zusätzlich mit Knochenzement auf der Resektionsfläche zu fixieren.

Wenn ein Implantat mit Führungs- und/oder Zentrierelementen, wie in Figur 9 dargestellt, versehen ist, müssen entsprechende Führungs - und/oder Zenrtierbohrungen, insbesondere mittels einer geeigneten Lehre, in die Resektionsfläche eingebracht werden. Diese sind beispielsweise so dimensioniert, dass sie die Führungs- und/oder Zentrierelemente möglichst spielfrei aufnehmen können. Bei der Implantation werden dann zuerst die Führungs- und/oder Zentrierelemente in die zugehörigen Bohrungen der Resektionsfläche eingeführt, wodurch Lage und/oder Richtung des Implantats auf der Resektionsfläche festgelegt sind, und dann das Implantat mit den Verankerungsstiften, für die keine Löcher vorgebohrt wurden, eingedrückt oder eingeschlagen.

### Bezugszeichenliste

- 1: Knochen
- 2: Spongiosa
- 3: Kortikalis
- 4: Bohrung
- 8: Scapula
- 9: Humerus
- 10: Implantat, Tibiaplatte
- 20: Verankerungsstift
- 21: Führungs- und/oder Zentrierzapfen
- 23: flächenhafte Verbindungsstruktur, wandartige Struktur
- 25: umbeschriebener Kreis eines Verankerungsstiftes
- 30: distale Femurkomponente
- 40: Femurkappe
- 41: Femur
- 42: Femurhals
- 43: Femurkopf
- 44: Resektionsfläche
- 50: Implantat
- 60: Humerusimplantat
- 61: Artikulationsfläche des Humerusimplantats
- 70: Glemoidimplantat
- 71: Artikulationsfläche des Glenoidimplantats
- 74: Drahtgitterstruktur

- d: Dicke eines Verankerungsstiftes, Durchmesser des umbeschriebenen Kreises eines Verankerungsstiftes
- dA: Flächenelement
- l: Länge eines Verankerungsstiftes
- h: Höhe der Wandartigen Struktur
- s: Abstand zweier Verankerungsstifte
- α: Winkel

## Patentansprüche

1. Implantat (10, 30, 40, 60, 70) zur Verankerung an einem Knochen (1, 41, 8, 9), mit einer Befestigungsseite, welche zur Anordnung benachbart zu einem Knochen vorgesehen ist, wobei auf der Befestigungsseite eine Mehrzahl sich von der Befestigungsseite weg erstreckender Verankerungsstifte (20) angeordnet sind, welche zum Verankern des Implantats im Knochen vorgesehen sind, wobei in unterschiedlichen Bereichen der Befestigungsseite des Implantats die Stiftanordnung und/oder die Geometrie der Stifte unterschiedlich gewählt sind, und bei welchem in verschiedenen Bereichen der Befestigungsseite des Implantats das Volumen der Verankerungsstifte (20) je Flächeneinheit (dA) der Befestigungsseite des Implantats unterschiedlich ist, **dadurch gekennzeichnet, dass** die Geometrie und/oder die Anordnung der Stifte (20) entsprechend des Anteils an knöchernem Gewebe am Gesamtgewebe des Knochenmaterials, dem gegenüberliegend der Bereich der Befestigungsseite zur Implantation vorgesehen ist, derart gewählt ist, dass in Bereichen eines relativ höheren Anteils knöchernen Gewebes gesamthaft weniger Gewebe durch die Verankerungsstifte verdrängt wird als in Bereichen eines relativ geringeren Anteils knöchernen Gewebes, wobei die Flächendichte der Verankerungsstifte wenigstens 3/cm² und höchstens 30/cm² beträgt.

2. Implantat gemäss Anspruch 1, **dadurch gekennzeichnet, dass** die Geometrie und/oder die Anordnung der Stifte (20) entsprechend des Anteils an knöchernem Gewebe am Gesamtgewebe des Knochenmaterials, dem gegenüberliegend der Bereich der Befestigungsseite vorgesehen ist, derart gewählt ist, dass jeweils im Wesentlichen gleich viel knöchernes Gewebe je Flächeneinheit der Befestigungsseite des Implantats verdrängt wird.

3. Implantat gemäss einem der vorstehenden Ansprüche, **dadurch gekennzeichnet, dass** die Geometrie und/oder die Anordnung der Verankerungsstifte (20) entsprechend des Anteils an knöchernem Gewebe am Gesamtgewebe des Knochenmaterials, dem gegenüberliegend der Bereich der Befestigungsseite vorgesehen ist, derart gewählt ist, dass in Bereichen eines relativ höheren Anteils knöchernen Gewebes das Volumen der Verankerungsstifte je Flächeneinheit der Befestigungsseite kleiner ist als in Bereichen eines relativ geringeren Anteils knöchernen Gewebes.

4. Implantat gemäss einem der vorstehenden Ansprüche, **dadurch gekennzeichnet, dass** die Verankerungsstifte (20) hinsichtlich ihrer Länge (I) und/oder hinsichtlich der Anzahl der Stifte je Flächeneinheit (dA) der Befestigungsseite und/oder hinsichtlich ihrer Querschnittsfläche variieren.

5. Implantat gemäss einem der vorstehenden Ansprüche, **dadurch gekennzeichnet, dass** an den dem Implantat benachbarten Enden der Verankerungsstifte (20) wenigstens zwei Verankerungsstifte durch eine wandartige Struktur (23) miteinander verbunden sind, welche sich einerseits von einem Verankerungsstift zum anderen Verankerungsstift erstreckt und sich andererseits um eine Höhe (h) von der Befestigungsseite zum abgewandten Ende der Verankerungsstifte hin erstreckt, wobei die Höhenerstreckung kleiner als die Länge der Verankerungsstifte ist.

6. Implantat gemäss einem der vorstehenden Ansprüche, **gekennzeichnet dadurch, dass** die Verankerungsstifte (20) wenigstens bereichsweise auf einem äquidistanten Raster, insbesondere auf einem Grundraster aus gleichseitigen Dreiecken, angeordnet sind.

7. Implantate gemäss einem der vorstehenden Ansprüche, **gekennzeichnet dadurch, dass** die Querschnitte der Verankerungsstifte (20) umbeschriebene Kreise aufweisen, deren Durchmesser (d) an der Basis der Stifte (20), d.h. am der Befestigungsseite des Implantats zugewandten Ende, wenigstens 0.5 mm und insbesondere wenigstens 1 mm betragen.

8. Implantat gemäss einem der vorstehenden Ansprüche, **dadurch gekennzeichnet, dass** die Längsachsen der Verankerungsstifte (20) im Wesentlichen parallel zueinander und bevorzugt im Wesentlichen senkrecht zu der Fläche der Befestigungsseite verlaufen.

9. Implantat gemäss einem der vorstehenden Ansprüche, **dadurch gekennzeichnet, dass** wenigstens ein Führungs- und/oder Zentrierelement (21) auf der Befestigungsseite angeordnet ist, welches sich bevorzugt weiter von der der Befestigungsseite weg erstreckt als der längste Verankerungsstift, und welches somit eine Positionierung des Implantats vor der Verankerung ermöglicht.

10. Implantat gemäss Anspruch 9, **dadurch gekennzeichnet, dass** wenigstens zwei und insbesondere genau zwei Führungs- und/oder Zentrierelemente (21) auf der Befestigungsseite angeordnet sind, welche sich bevorzugt weiter von der Befestigungsseite weg erstrecken als der längste Verankerungsstift, und welche somit eine Positionierung des Implantats nach Lage und Richtung vor der Verankerung ermöglichen.

11. Implantat gemäss einem der vorstehenden Ansprüche **dadurch gekennzeichnet, dass** die Länge der Verankerungsstifte (20) und/oder die Anzahl der Verankerungsstifte pro Flächeneinheit der Befestigungsseite des Implantats und/oder die Querschnittsfläche der einzelnen Verankerungselemente vom Rand der Befestigungsseite des Implantats zur Mitte der Flächen hin zunehmen.

12. Implantat gemäss einem der vorstehenden Ansprüche als proximales Tibiaimplantat, insbesondere als Tibiakomponente einer Kniegelenksprothese, oder als distale Femurkomponente einer Kniegelenksprothese.

13. Implantat gemäss einem der vorstehenden Ansprüche als proximale Femurkomponente für ein Hüftgelenk, insbesondere zur Anordnung auf dem Femurkopf, und insbesondere mit einer Metall-Artikulationsfläche, oder als Acetabulumkomponente einer Hüftgelenksprothese.

14. Implantat gemäss einem der vorstehenden Ansprüche als Bestandteil einer Schultergelenksprothese zur Verankerung an der Scapula oder zur Verankerung am proximalen Humerus.

15. Implantat gemäss einem der vorstehenden Ansprüche als Bestandteil eines intervertebralen Implantats.

## Claims

1. An implant (10, 30, 40, 60, 70) for anchorage to a bone (1, 41, 8, 9) having a fastening side which is provided for arrangement adjacent to a bone, wherein a plurality of anchorage pins (20) are arranged at the fastening side which extend away from the fastening side and which are provided for the anchorage of the implant in the bone, wherein the pin arrangement and/or the geometry of the pins is/are selected differently in different regions of the fastening side of the implant and wherein the volume of the anchorage pins (20) per unit of area (dA) of the fastening side of the implant is different in different regions of the fastening side, **characterized in that** the geometry and/or the arrangement of the pins (20) is/are selected in accordance with the proportion of bony tissue in the total tissue of the bone material, opposite to which the region of the fastening side is provided for the implantation, such that in total less tissue is displaced by the anchorage pins in regions of a relatively higher proportion of bony tissue than in regions of a relatively lower proportion of bony tissue, wherein the surface density of the anchorage pins amounts to at least 3/cm² and at most 30/cm².

2. An implant in accordance with claim 1, **characterized in that** the geometry and/or the arrangement of the pins (20) is/are selected in accordance with the proportion of bony tissue in the total tissue of the bone material, opposite which the region of the fastening side is provided, such that in each case substantially the same amount of bony tissue is displaced per unit of area of the fastening side of the implant.

3. An implant in accordance with one of the preceding claims, **characterized in that** the geometry and/or the arrangement of the anchorage pins (20) is/are selected in accordance with the proportion of bony tissue in the total tissue of the bone material, opposite which the region of the fastening side is provided, such that the volume of the anchorage pins per unit of area of the fastening side is smaller in regions of a relatively higher proportion of bony tissue than in regions of a relatively lower proportion of bony tissue.

4. An implant in accordance with any one of the preceding claims, **characterized in that** the anchorage pins (20) vary with respect to their length (I) and/or with respect to the number of pins per unit of area (dA) of the fastening side and/or with respect to their cross-sectional area.

5. An implant in accordance with any one of the preceding claims, **characterized in that** at least two anchorage pins (20) are connected to one another by a wall-like structure (23) at the ends of the anchorage pins (20) adjacent to the implant, said wall-like structure extending from one anchorage pin to the other anchorage pin, on the one hand, and extending by a height (h) from the fastening side toward the remote end of the anchorage pins, on the other hand, with the vertical extent being smaller than the length of the anchorage pins.

6. An implant in accordance with any one of the preceding claims, **characterized in that** the anchorage pins (20) are arranged at least regionally on an equidistant grid, in particular on a base grid of equilateral triangles.

7. An implant in accordance with any one of the preceding claims, **characterized in that** the cross-sections of the anchorage pins (20) have circumscribed circles whose diameters (d) amount to at least 0.5 mm and in particular at least 1 mm at the base of the pins (20), i.e. at the end facing the fastening side of the implant.

8. An implant in accordance with any one of the preceding claims, **characterized in that** the longitudinal axes of the anchorage pins (20) extend substantially parallel to one another and preferably perpendicular to the surface of the fastening side.

9. An implant in accordance with any one of the preceding claims, **characterized in that** at least one guide member and/or centering member (21) is arranged on the fastening side, preferably extends further away from the fastening side than the longest anchorage pin and thus enables a positioning of the implant before the anchorage.

10. An implant in accordance with claim 9, **characterized in that** at least two guide members and/or centering members (21), and in particular precisely two guide members and/or centering members, are arranged at the fastening side, preferably extend further away from the fastening side than the longest anchorage pin and thus enable a positioning of the implant by position and direction before the anchorage.

11. An implant in accordance with any one of the preceding claims, **characterized in that** the length of the anchorage pins (20) and/ or the number of the anchorage pins per unit of area of the fastening side of the implant and/or the cross-sectional surface of the individual anchorage elements increases/increase from the rim of the fastening side of the implant toward the center of the surfaces.

12. An implant in accordance with any one of the preceding claims as a proximal tibial implant, in particular as a tibial component of a knee joint prosthesis, as a distal femoral component of a knee joint prosthesis.

13. An implant in accordance with any one of the preceding claims as a proximal femoral component for a hip joint, in particular for arrangement on the femoral head, and in particular having a metal articulation surface, or as an acetabulum component of a hip joint prosthesis.

14. An implant in accordance with any one of the preceding claims as a component of a shoulder joint prosthesis for anchorage to the scapula or for anchorage to the proximal humerus.

15. An implant in accordance with any one of the preceding claims as a component of an intervertebral implant.

## Revendications

1. Implant (10, 30, 40, 60, 70) pour l'ancrage sur un os (1, 41, 8, 9), comprenant un côté fixation qui est prévu pour être agencé au voisinage d'un os, et une pluralité de tiges d'ancrage (20) s'étendant en éloignement du côté fixation sont agencées sur le côté fixation et sont prévues pour ancrer l'implant dans l'os, et l'agencement des tiges et/ou la géométrie des tiges est choisi(e) de manière différente dans des zones différentes du côté fixation, et pour les tiges, dans différentes zones du côté fixation de l'implant, le volume des tiges d'ancrage (20) par unité de surface (dA) du côté fixation de l'implant est différent, **caractérisé en ce que** la géométrie et/ou l'agencement des tiges (20) est choisi(e) en correspondance de la proportion de tissu osseux dans la totalité des tissus du matériau osseux à l'opposé duquel est prévue la zone du côté fixation pour l'implantation, de telle manière que dans des zones où la proportion de tissu osseux est relativement élevée une quantité totale de tissu refoulé par les tiges d'ancrage est plus faible que dans les zones où la proportion de tissu osseux est relativement plus faible, et la densité surfacique des tiges d'ancrage s'élève à au moins 3/cm² et au plus à 30/cm².

2. Implant selon la revendication 1, **caractérisé en ce que** la géométrie et/ou l'agencement des tiges (20) est choisi(e) en correspondance de la proportion de tissu osseux dans la totalité des tissus du matériau osseux à l'opposé duquel est prévue la zone du côté fixation, de telle manière que la quantité de tissu osseux refoulé par unité de surface du côté fixation de l'implant est respectivement sensiblement égale.

3. Implant selon l'une des revendications précédentes, **caractérisé en ce que** la géométrie et/ou l'agencement des tiges d'ancrage (20) est choisi(e) en correspondance de la proportion de tissu osseux dans la totalité des tissus du matériau osseux à l'opposé duquel est prévue la zone de fixation, de telle manière que le volume des tiges d'ancrage par unité de surface du côté fixation est plus faible dans des zones avec une proportion relativement élevée de tissu osseux que dans des zones avec une proportion relativement plus faible de tissu osseux.

4. Implant selon l'une des revendications précédentes, **caractérisé en ce que** les tiges d'ancrage (20) varient quant à leur longueur (1) et/ou quant au nombre des tiges par unité de surface (dA) du côté fixation et/ou quant à leur surface de section transversale.

5. Implant selon l'une des revendications précédentes, **caractérisé en ce qu'**aux extrémités, voisines de l'implant, des tiges d'ancrage (20) au moins deux tiges d'ancrage sont reliées l'une à l'autre par une structure analogue à une paroi (23), qui s'étend d'une part depuis une tige d'ancrage jusqu'à l'autre tige d'ancrage et qui s'étend d'autre part sur une hauteur (h) depuis le côté fixation vers l'extrémité détournée des tiges d'ancrage, dans lequel l'extension en hauteur est inférieure à la longueur des tiges d'ancrage.

6. Implant selon l'une des revendications précédentes, **caractérisé en ce que** les tiges d'ancrage (20) sont agencées au moins par zones sur une trame équidistante, en particulier sur une trame de base de triangles équilatéraux.

7. Implant selon l'une des revendications précédentes, **caractérisé en ce que** les sections transversales des tiges d'ancrage (20) présentent des cercles circonscrits dont le diamètre (d) au niveau de la base des tiges (20) c'est-à-dire à l'extrémité tournée vers le côté fixation de l'implant, est d'au moins 0,5 mm et en particulier d'au moins 1 mm.

8. Implant selon l'une des revendications précédentes, **caractérisé en ce que** les axes longitudinaux des tiges d'ancrage (20) s'étendent sensiblement parallèlement les uns aux autres et de préférence sensiblement perpendiculairement à la surface du côté fixation.

9. Implant selon l'une des revendications précédentes, **caractérisé en ce qu'**au moins un élément de guidage et/ou de centrage (21) est agencé sur le côté fixation, qui s'étend de préférence en éloignement du côté fixation plus loin que la plus longue tige d'ancrage, et qui permet ainsi un positionnement de l'implant avant l'ancrage.

10. Implant selon la revendication 9, **caractérisé en ce qu'**au moins deux et en particulier exactement deux éléments de guidage et/ou de centrage (21) sont agencés sur le côté fixation, qui s'étendent de préférence en éloignement du côté fixation plus loin que la plus longue tige d'ancrage, et qui permettent ainsi un positionnement de l'implant en situation et en direction avant l'ancrage.

11. Implant selon l'une des revendications précédentes, **caractérisé en ce que** la longueur des tiges d'ancrage (20) et/ou le nombre des tiges d'ancrage par unité de surface du côté fixation de l'implant et/ou la surface de section transversale des éléments d'ancrage individuels augmente depuis la bordure du côté fixation de l'implant vers le milieu des surfaces.

12. Implant selon l'une des revendications précédentes, à titre d'implant proximal du tibia, et en particulier à titre de composant tibial d'une prothèse de l'articulation du genou, ou bien à titre de composant distal du fémur d'une prothèse de l'articulation du genou.

13. Implant selon l'une des revendications précédentes, à titre de composant proximal du fémur pour une articulation de la hanche, en particulier pour l'agencement sur la tête du fémur, et en particulier avec une surface d'articulation en métal, ou à titre de composant acétabulaire d'une prothèse de l'articulation de la hanche.

14. Implant selon l'une des revendications précédentes à titre de partie constitutive d'une prothèse articulaire de l'épaule destinée à l'ancrage sur l'os scapulaire, ou destinée à l'ancrage sur l'humérus proximal.

15. Implant selon l'une des revendications précédentes à titre de partie constitutive d'un implant intervertébral.
